# EUROPEAN PATENT APPLICATION

(11) **EP 1 380 305 A1**
(43) Date of publication of application: **14.01.2004**
(21) Application number: 03015227.6
(22) Date of filing: 05.07.2003
(51) Int. Cl.: A61K 41/00

(54) **Photodynamic therapy for the enhancement of vascular permeability**

(30) Priority: 11.07.2002 US 193417
(71) Applicant: HEALTH RESEARCH, INC., Buffalo New York 14263 (US)
(72) Inventor: Henderson, Barbara W., Buffalo, New York 14214 (US); Oseroff, Allan R., Buffalo, New York 14216 (US); Bellnier, David A., Buffalo, New York 14214 (US)
(74) Representative: Weber, Dieter, Dr.

(57) **Abstract**

A method for treating aberrant tissue in an animal that includes the following steps. A photosensitive agent is introduced into the blood stream of an animal that has better selectivity for the aberrant tissue than normal tissue to obtain aberrant tissue containing the photosensitizing agent. The aberrant tissue containing the photosensitizing agent is exposed to light to activate the photosensitizing agent to increase permeability of vasculature in the aberrant tissue; and a treating agent is introduced into the bloodstream of the animal, which treating agent passes through the vasculature having increased permeability into the aberrant tissue to treat the aberrant tissue.

## Description

### BACKGROUND OF THE INVENTION

Photodynamic therapy (PDT) is a relatively new modality for the treatment of various types of solid tumors. Many porphyrins and related photosensitive compounds, e.g. PHOTOFRIN® hematoporphyrin derivative (HpD), HPPH, BPD, etc., demonstrate the ability to selectively accumulate in neoplastic tissue after intravenous injection and sensitize the tissue to light. Activation of the photosensitive agent by visible light, usually delivered by a laser through fiber optics, results in the generation of cytotoxic agents. It is currently believed that the production of singlet oxygen, formed from molecular oxygen by the transfer of energy directly or indirectly from the activated photosensitizer, is primarily responsible for tumor destruction.

In 1978, Dougherty reported that a combination of hematoporphyrin derivative (HpD) and light was effective in causing partial or complete tumor necrosis in 111 of 113 tumors in 25 patients. Since then, PDT with PHOTOFRIN®, a purified HpD, has been approved in Canada for bladder and esophageal cancer; in the Netherlands and France for early- and advanced-stage esophageal cancer; in Japan for early-stage lung, esophageal, gastric and cervical cancer; and in the United States for advanced-stage esophageal and both early- and late-stage lung cancers. Thousands of patients worldwide have now been treated with PDT for a multitude of tumors accessible to light, including skin, lung, bladder, head and neck, breast, and esophageal cancers.

PDT exerts its antitumor effects via the interplay of several mechanisms: direct phototoxicity to the tumor cells, destruction of the vasculature, and an inflammatory, as well as possibly cell mediated immune response. Dose-dependent vascular PDT effects can include acute vessel constriction, followed by dilation and leakiness, and eventual thrombosis and occlusion. Despite the above three-pronged attack, tumor cure by PDT often requires doses that compromise treatment selectivity. Even when selectivity is high, tumor regrowth often occurs.

Chemo- and immuno-therapy have their own limitations, one of which is the fact that the tumor vasculature presents a barrier to the effective delivery of agents to the target sites. This is especially true for macromolecular drug delivery systems designed to lower toxicity or increase therapeutic efficacy and selectivity. These may include monoclonal antibody conjugates, soluble polymeric and nanoparticle carriers ([immuno]liposomes and microspheres), gene vectors and even immune effector cells. In order to reach the target (tumor) cell, a systemically administered molecule or particle has to distribute throughout the vascular space and transport across the microvascular wall and through the interstitial space. Unfortunately, the highly heterogeneous intrinsic permeability of tumor vessels for both small and large molecules is only sufficient for drug transport at the tumor-host interface. Therefore, therapeutic modalities to increase the site-specific delivery of therapeutic agents in a more homogeneous manner would be desirable.

Both chemical and physical approaches have been tried with some success to improve drug delivery to tumors. These include the administration of vasoactive agents, ionizing radiation and local hyperthermia. Such approaches have not, however been as successful as desired.

### BRIEF DESCRIPTION OF THE INVENTION

In accordance with the invention a method is therefore provided for treating aberrant tissue in an animal. The method includes the following steps:
A photosensitive agent is introduced into the blood stream of animal which photosensitizing agent has better selectivity for the aberrant tissue than normal tissue to obtain aberrant tissue containing the photosensitizing agent;
The photosensitizing agent is exposed to light to activate the photosensitizing agent to increase permeability of vasculature in the aberrant tissue; and
a treating agent is introduced into the bloodstream of the animal, which treating agent passes through said vasculature having increased permeability into the aberrant tissue to treat the aberrant tissue.

The aberrant tissue is usually hyperproliferative tissue, i.e. tissue that grows more rapidly than is consistent with homeostasis. Such hyperproliferative tissue is usually tumor tissue but may be other tissues such as hyperproliferative vasculature found in age related macular degeneration. The treating agent is usually a chemotherapeutic agent that causes necrosis of the aberrant tissue. Examples of such chemotherapeutic agents include adriamycin which may be conjugated with a polymer such as N-(2-hydroxypropyl)methacrylamide) to reduce toxicity and increase residence time. The chemotherapeutic agent may be liposome encapsulated to increase transfer across cellular membranes.

The photosensitizing agent is any photosensitizing agent that preferentially collects in the hyperproliferative tissue. Examples of such photosensitizing agents include porphyrin derivatives such as: the hematoporphyrin derivative PHOTOFRIN®; HPPH (3-(1'-hexyloxy)ethyl-3-devinyl-pyropheophorbide-a), also named (2-[1-hexyl-oxyethyl]-2-devinyl pyropheophobide-a; and BPD benzyl porphyrin derivative (hydro-monobenzylporphyrin as described in U.S. Patent 4,883,790 incorporated herein by reference.

### BRIEF DESCRIPTION OF THE DRAWINGS

The patent application file contains at least one drawing executed in color. Copies of this patent application with the color drawing will be provided by the Office upon request and payment of the necessary fee.
Figure 1 is a set of MR images of Colo-26 tumors taken 30 minutes after P-PDT to the tumor on the left of each image. The tumor on the right of each image shows an untreated control.
Figure 2 is a bar graph showing SL-DOX uptake in P-PDT treated tumors (black bars) compared with untreated controls (grey bars). The tumors are named on the x-axis.
Figure 3 is a graph showing Colo-26 tumor response to SL-DOX delivered with and without prior P-PDT.
Figure 4 is a bar graph showing SL-DOX uptake in Colo-26 tumor after P-PDT using PHOTOFRTN® hematoporphyrin derivative.

### DETAILED DESCRIPTION OF THE INVENTION

In accordance with the present invention, we have found that the uptake of systemically administered macromolecular therapeutic agents can be significantly enhanced in hyperproliferative tissue, e.g. a tumor, with a PDT approach that causes increased vascular leakiness, thus increasing the therapeutic effectiveness of those agents. We have termed this PDT approach "permeabilizing" PDT or P-PDT. The invention is therefore a method or process to enhance the egress of therapeutic agents into and through walls of vasculature and, therefore, increase their uptake in diseased tissues such as malignant tumors.

In the above, PDT is applied using appropriate photosensitizers and/or photosensitizer combinations that can be activated locally by light at photosensitizer/light doses that induce vascular leakiness. This is followed by the systemic administration of the therapeutic agent. The P-PDT induced vascular leakiness allows the therapeutic agents to leave the vasculature and distribute into hyperproliferative tissue, e.g. the tumor bed, in higher concentrations than achievable without prior permeabilizing PDT. This enhances the access of the therapeutic agent to its cellular target, thereby enhancing therapeutic effectiveness and selectivity.

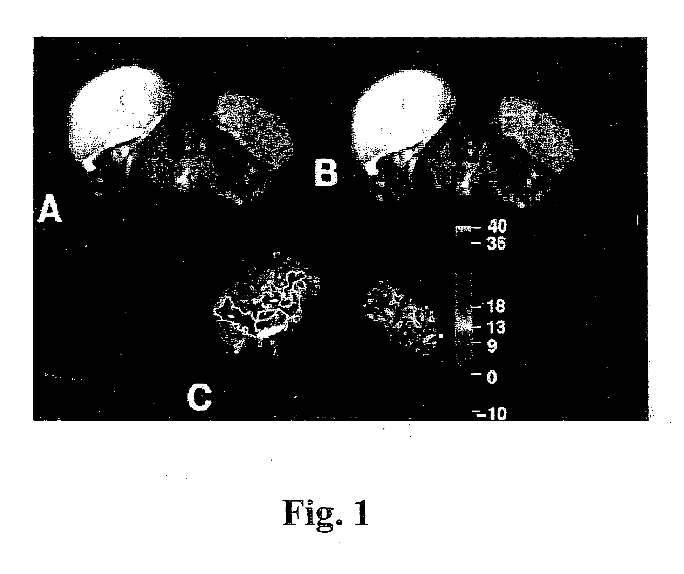

Figure 1 illustrates the response of the vasculature in murine Colo-26 tumors to P-PDT. The figure shows MR images of a set of Colo-26 tumors acquired 30 min after P-PDT to the tumor on the left of each image. A shows an axial T2 weighted image; B shows an axial T2 weighted image acquired during carbogen breathing by the animal; and C shows an IMR image with mapping of percent pixel intensity change during carbogen breathing. In this case, P-PDT involved i.v. injection of 0.4 µmol/kg HPPH (2-[1-hexyl-oxyethyl]-2-devinyl pyropheophoribde-a), followed 24 h later by i.v. injection of 6 µmol/kg VBBO (Victoria Blue-BO), followed 3 h later by local exposure of the tumor to 500 J/cm² of light at a wavelength of 619 nm. In Frame A, one can observe that the T2 weighted MRI image of the P-PDT treated tumor is markedly brighter than the control, which is believed to be due to increased water content because of enhanced edema and permeability in that tumor. Image intensity is further heightened by carbogen breathing as shown in Frame B of Figure 1. The false color image of Frame C quantifies image intensity and shows large increases in image intensity (up to 40%), especially in the treated tumor. Changes in signal intensity originate with the magnetic susceptibility of hemoglobin and are dependent on its oxygenation status. Oxyhemoglobin is diamagnetic, while deoxyhemoglobin is paramagnetic. The magnetic susceptibility differences between deoxyhemoglobin and surrounding tissue water create local field gradients that cause phase dispersion of nearby water protons, resulting in a loss of MR signal intensity. Therefore, increases in signal intensity in response to carbogen breathing reflect changes in tumor oxygenation that may stem from increased blood oxygenation, perfusion and/or blood flow.

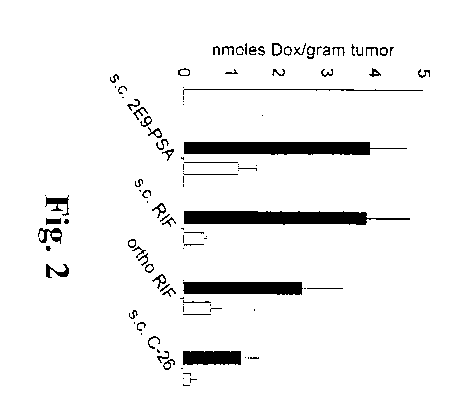

Figure 2 illustrates that P-PDT can improve the tumor uptake of macromolecular complexes used for drug delivery. Sterically stabilized liposomes encapsulating adriamycin also known as doxorubicin (SL-DOX), one of the most active anti-cancer drugs in current clinical use, was used. The sterically stabilized liposomes were about 100 nm in diameter. This delivery system has been studied extensively in other methods because it provides for increased tumor uptake and efficacy compared with free DOX with less toxicity. The system is representative of encapsulated treatment compounds that are relatively large and thus often have difficulty in passing cell membranes. P-PDT was carried out on one tumor of a set of two tumors in each of four different tumor systems, a human lung tumor xenograft (2E9-PSA), the murine RIF tumor (implanted either ectopically [s.c. RIF] or orthotopically [ortho RIF]), and the murine Colo-26 tumor. In all cases, the P-PDT treated tumor accumulated significantly more DOX than the untreated control tumors. The increase in tumor localization ranged from ∼3 to 10 times that of controls. (See Figure 2.)

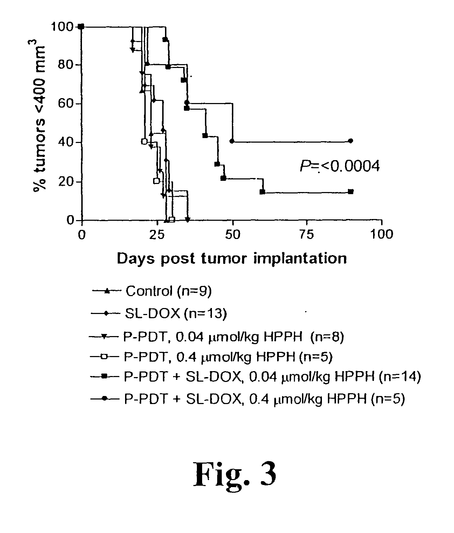

Not only did P-PDT enhance SL-DOX tumor uptake, it also significantly enhanced the anti-tumor efficacy of DOX. This is shown in Fig. 3, where SL-DOX was injected at a single dose of 2.5 mg/kg into mice carrying a single Colo-26 tumor and exposed to P-PDT. P-PDT was given as described at two different HPPH doses, 0.4 and 0.04 µmol/kg. Controls received P-PDT only or SL-DOX only. Treatment commenced on day 11 after tumor implantation (when the tumors were 6-8 mm diameter). Control treatments, both SL-DOX and P-PDT only, showed minimal anti-tumor effects. Among the 14 animals in the P-PDT + SL-DOX group at the low HPPH dose, there were 2 mice cured at 90 days post treatment, with a doubling of the tumor regrowth time in the remaining animals *versus* controls. These results, which were highly significant, are almost identical to those obtained by Huang et a1. (Cancer Res., 52:6774-6781, 1992) where a single dose of 8 mg/kg SL-DOX and two local hyperthermia treatments were necessary to achieve them in the same tumor model.

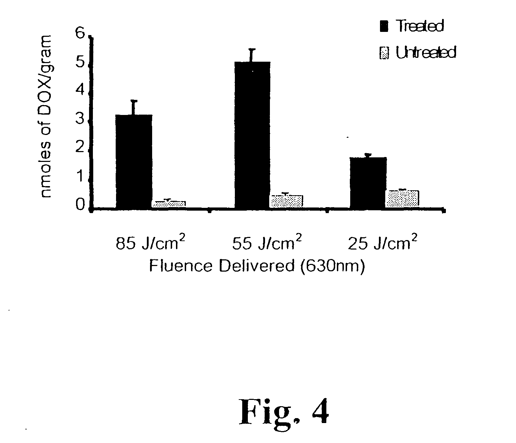

Figure 4 is a bar graph showing SL-DOX uptake in Colo-26 tumor after P-PDT using PHOTOFRIN® hematoporphyrin derivative at 5 mg/kg injected dose. Tumors were exposed to a range of subcurative doses of light at 630 nm and 75 mW/cm². SL-DOX was injected i.v. immediately after light exposure. Tumors were harvested five hours after drug injection and assayed for SL-DOX content. The black bars show tumors treated with PHOTOFRIN®. The gray bars are controls showing low DOX uptake in tumors untreated with PHOTOFRIN®. It is clear that PDT treatment enhanced SL-DOX uptake.

## Claims

1. A porphyrin derivative photosensitizer for treating aberrant tissue in an animal by introducing the photosensitizer into the blood stream of animal which photosensitizer has better selectivity for the aberrant tissue than normal tissue to obtain aberrant tissue containing the photosensitizer, and exposing the aberrant tissue containing the photosensitizing agent to light to activate the photosensitizing agent to increase permeability of vasculature in the aberrant tissue, **characterized in that** subsequent to activating the photosensitizing agent to increase permeability, a treating agent is introduced into the bloodstream of the animal, which treating agent passes through said vasculature having increased permeability into the aberrant tissue to treat the aberrant tissue.

2. The photosensitizer of claim 1 **characterized in that** the aberrant tissue is hyperproliferative tissue and the treating agent causes necrosis of the aberrant tissue.

3. The photosensitizer of claim 2 **characterized in that** the aberrant tissue is tumor tissue.

4. The photosensitizer of claim 2 **characterized in that** the aberrant tissue is hyperproliferative vasculature.

5. The photosensitizer of claim 1 **characterized in that** the treating agent is a chemotherapeutic agent.

6. The photosensitizer of claim 1 **characterized in that** the porphyrin derivative is hematoporphyrin derivative.

7. The photosensitizer of claim 1 **characterized in that** the porphyrin derivative is HPPH.

8. The photosensitizer of claim 1 **characterized in that** the porphyrin derivative is benzoporphyrin derivative.

9. The photosensitizer of claim 1 **characterized in that** the porphyrin derivative is a chlorine derivative.

10. The photosensitizer of claim 1 **characterized in that** the porphyrin derivative is a bacteriochlorin derivative.

11. The photosensitizer of claim 1 **characterized in that** the porphyrin derivative is a purpurin derivative.
